(19) Europäisches Patentamt / European Patent Office / Office européen des brevets

(11) **EP 3 419 515 B1**

(12) **EUROPÄISCHE PATENTSCHRIFT**

(45) Veröffentlichungstag und Bekanntmachung des Hinweises auf die Patenterteilung:
**22.07.2020   Patentblatt 2020/30**

(51) Int Cl.:
**A61B 5/0225** *(2006.01)*        **A61B 5/0235** *(2006.01)*

(21) Anmeldenummer: **16711116.0**

(86) Internationale Anmeldenummer:
**PCT/AT2016/050035**

(22) Anmeldetag: **22.02.2016**

(87) Internationale Veröffentlichungsnummer:
**WO 2017/143366 (31.08.2017 Gazette 2017/35)**

(54) **VERFAHREN UND MESSSYSTEM ZUR KONTINUIERLICHEN BESTIMMUNG DES INTRA-ARTERIELLEN BLUTDRUCKES**

METHOD AND MEASURING SYSTEM FOR CONTINUOUSLY DETERMINING THE INTRA-ARTERIAL BLOOD PRESSURE

PROCÉDÉ ET SYSTÈME DE MESURE POUR LA DÉTERMINATION CONTINUE DE LA PRESSION SANGUINE INTRA-ARTÉRIELLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**02.01.2019   Patentblatt 2019/01**

(73) Patentinhaber: **CNSystems Medizintechnik AG**
**8020 Graz (AT)**

(72) Erfinder:
• **FORTIN, Jürgen**
  **8043 Graz (AT)**
• **FORTIN, Andrea**
  **8043 Graz (AT)**

• **POPP, Klaus**
  **4580 Windischgarsten (AT)**

(74) Vertreter: **Babeluk, Michael**
  **Florianigasse 26/3**
  **1080 Wien (AT)**

(56) Entgegenhaltungen:
WO-A2-2007/028107     AT-B- 412 613
JP-A- H0 924 029      JP-A- H06 125 882
JP-A- H07 308 297     JP-A- H10 312 241
JP-B2- 3 486 976      KR-A- 20100 042 566
US-A- 3 229 685       US-A- 5 218 966

EP 3 419 515 B1

## Beschreibung

Die Erfindung betrifft ein Verfahren und ein Messsystem zur kontinuierlichen Bestimmung des intra-arteriellen Blutdruckes an zwei Fingern einer Hand, mit Hilfe eines Doppelfingersensors. Dieser weist ein plethysmographisches System, mit zumindest einer Lichtquelle, zumindest einem Lichtaufnehmer bzw. Lichtdetektor und zumindest einer aufblasbaren Manschette auf. Weiters ist ein Druckerzeugungssystem mit zumindest einem mit Hilfe des plethysmographischen Systems in Echtzeit geregelten Ventil zur Erzeugung eines Druckes in der Manschette vorgesehen, der im Wesentlichen dem intra-arteriellen Blutdruck im Finger entspricht.

Die kontinuierliche nicht-invasive Messung des Blutdruckes stellt bis heute eine große Herausforderung an die Messtechnik dar. Am Markt beginnt sich die sog. "Vascular Unloading Technique" durchzusetzen, die auf eine Publikation von Penáz (Digest of the 10th International Conference on Medical and Biological Engineering 1973 Dresden) zurückgeht und durch verschiedenste Elemente verbessert wurde.

Bei der Vascular Unloading Technique wird zunächst ein Finger durchleuchtet und so der pulsatile (pulsförmige) Blutfluss im Finger bestimmt. Dieses Verfahren wird auch Photoplethysmographie (PPG) genannt und wird üblicherweise mit Hilfe von einer oder mehreren Leuchtdioden LED's, die mit einer oder mehreren Wellenlängen arbeiten, sowie mit Hilfe von einer oder mehreren lichtempfindlichen Empfängerdioden (Fotodioden) erzeugt.

Ein Regelungssystem hält nun diesen registrierten Fluss und somit das resultierende PPG-Signal (Volumssignal v(t) konstant, in dem ein Gegendruck in einer Manschette (Cuffpressure $p_c(t)$ am Finger aufgebracht wird. Dieser Gegendruck $p_c(t)$ wird dabei von einem schnellen Ventil oder Ventilsystem erzeugt, das von einer Pumpe versorgt wird. Die diesbezügliche Ansteuerung des Ventils bez. des Ventilsystems wird von dieser Regelungseinheit durchgeführt, die vorzugweise mit einem Microcomputer realisiert wird. Das wichtigste Eingangssignal ist dabei das PPG-Signal v(t), bei neuartigen Regelsystemen wird auch zusätzlich der Manschettendruck $p_c(t)$ als Eingangsvariable verwendet. Der für die Konstanthaltung des Flusses bzw. des PPG-Signales v(t) notwendige Druck $p_c(t)$ entspricht nun dem intra-arteriellen Blutdruck $p_a(t)$.

Dafür ist es notwendig, dass sich der Manschettendruck $p_c(t)$ mindestens so schnell verändern lässt, wie sich auch der intra-arterielle Blutdruck $p_a(t)$ ändert, damit die Echtzeitbedingung erfüllt ist. Damit diese Regelbedingung also vollständig eingehalten werden kann, muss der Manschettendruck $p_c(t)$ den intra-arteriellen Blutdruck $p_a(t)$ im sogenannten Zeitbereich als auch im sogenannten Frequenzbereich nachbilden können. Die obere Grenzfrequenz von $p_a(t)$ und somit die höchste Druckänderungsgeschwindigkeit liegt oberhalb von zumindest 20Hz, was für das Drucksystem durchaus eine große Herausforderung darstellt. Daraus folgt, dass sich die Druckerzeugung durch ein Ventil bzw. Ventilsystem in der unmittelbaren Nähe der Manschette befinden muss. Bei zu langen Luftleitungen droht der Verlust dieser Grenzfrequenzbedingung wegen der Tiefpasswirkung der Schläuche. Bei allen bisher bekannten kommerziell verfügbaren sowie bei den veröffentlichten Geräten und Verfahren ist daher das Ventil / Ventilsystem bzw. das Druckerzeugungssystem am distalen Unterarm, proximal des Handgelenkes - entweder oberhalb oder unterhalb- angebracht.

Das Anbringen des Druckerzeugungssystems am distalen Unterarm, proximal des Handgelenkes hat wesentliche Nachteile: Diese Stelle wird gerne für intravenöse Zugänge verwendet und auch der intra-arterielle Zugang am distalen Ende des Radius sollte für Notfälle frei sein. Diese Zugänge wären durch die Druckerzeugung und dessen Befestigung blockiert. Außerdem kann im Betrieb das System verrutschen bzw. kippen. Dies kann sich nachteilig auf den Sitz der Sensoren auswirken. Der Sitz der Sensoren würde sich außerdem verbessern, wenn sich der zu messende Finger bzw. die entsprechende Hand in einer gewissen Ruhelage befindet.

Zahlreiche Veröffentlichungen betreffend der Vascular Unloading Technique sind bekannt: Die US 4,406,289 (Wesseling et al.) beschreibt ein mechanisches Ventil, das den Gegendruck in der Fingermanschette mit der gewünschten Genauigkeit erzeugt, wenn es mit einer linearen Pumpe versorgt wird. Das Ventil ist in einem Gehäuse am distalen Unterarm untergebracht und versorgt so die Fingermanschette über einen kurzen Schlauch mit dem Druck $p_c(t)$.

Die US 4,524,777 (Kisioka et al.) beschreibt ein Druckerzeugungssystem für die Vascular Unloading Technique. Dabei wird zunächst ein konstanter Manschettendruck $P_c$ mit einer linearen Pumpe erzeugt, der mit Druckschwankungen $\Delta p_c(t)$ überlagert wird, die aus einem parallel geschalteten "Shaker" bzw. einem " Driving Actuator" kommen.

Die US 4,726,382 (Boehmer et al.) beschreibt eine Fingermanschette für die Vascular Unloading Technique, die Schlauchanschlüsse für die Versorgung mit dem Manschettendruck $p_c(t)$ besitzt. Die Länge der Luftschläuche reichen bis zum (nicht gezeigten) Druckerzeugungssystem, das auf dem distalen Unterarm befestigt ist.

Die WO 2000/059369 (Fortin et al.) beschreibt ebenfalls ein Druckerzeugungssystem für die Vascular Unloading Technique. Das Ventilsystem besteht aus einem separaten Einlass- und einem separaten Auslassventil. Während bei den Patentschriften US 4,406,289 und US 4,524,777 eine relativ lineare Proportionalpumpe verwendet werden muss, erlaubt dieses System die Verwendung von einfachen kostengünstigen Pumpen, denn die störenden Oberwellen werden durch die Anordnung der Ventile eliminiert. Weiters kann der Energieverbrauch der einfachen Pumpe durch das Ventilprinzip we-

Die WO 2004/086963 (Skrabal et al.) beschreibt ein System für die Vascular Unloading Technique, bei dem in einem Finger kontinuierlich der Blutdruck bestimmt werden kann, während im benachbarten Finger eine Qualitätskontrolle ("Watch Dog") vorgenommen wird. Nach einer Zeit wechselt das System automatisch den "Messfinger" mit dem "Überwachungsfinger".

Die WO 2005/037097 (Fortin et al.) beschreibt ein Regelungssystem für die Vascular Unloading Technique, bei der mehrere ineinander verflochtene Regelkreise für die Erzeugung von hochfrequenten Druckänderungen bis hin zur Langzeitstabilität für Genauigkeit sorgen.

Die WO 2010/050798 (Guelen et al.) beschreibt im Wesentlichen ein am distalen Unterarm befestigtes Druckerzeugungssystem ("Frontend") mit nur einem Ventil, an dem eine Fingermanschette für die Vascular Unloading Technique angebracht werden kann.

Die WO 2011/045138 (Langewouters et al.) beschreibt ein Druckerzeugungssystem für die Vascular Unloading Technique mit dem - ähnlich wie bei der WO 2000/059369 - die Energieaufnahme der Pumpe reduziert sowie die Oberwellen eliminiert werden.

Die WO 2011/051819 (Fortin et al.) beschreibt eine vollständig in digitaler Elektronik implementierte neue Art der Vascular Unloading Technique, um eine weitere Verbesserung der Stabilität sowie weitere Miniaturisierungen zu ermöglichen.

Die WO 2011/051822 (Fortin et al.) beschreibt ein Verfahren für die Vascular Unloading Technique, bei dem die gemessenen Signale v(t) und $p_c(t)$ derartig verarbeitet werden, um die langfristige Stabilität zu erhöhen und um weitere hämodynamische Parameter daraus zu gewinnen. Insbesondere werden ein Verfahren zur Elimination von vasomotorischen Veränderungen der Fingerarterien sowie eine Methode zur Bestimmung des Herzzeitvolumens beschrieben.

Die WO 2012/032413 (Huber et al.) beschreibt neuartige Fingersensoren, die ein Wegwerfteil besitzen.

Weitere dem Stand der Technik entsprechende Messsysteme sind z.B. auch in AT412613B und US5218966A beschrieben.

Aufgabe der Erfindung ist es, ausgehend von einem Messsystem zur kontinuierlichen, nicht-invasiven Bestimmung des intra-arteriellen Blutdruckes der eingangs beschriebenen Art Verbesserungen vorzuschlagen, die den Tragekomfort für den Patienten erhöhen, und bei längeren Messzeiten ermüdende Handstellungen vermeiden.

Diese Aufgabe wird dadurch gelöst, dass das Messsystem ein Gehäuse mit einer Oberfläche aufweist, die als Auflagefläche für den zumindest einen Finger und die angrenzenden Bereiche der Handinnenfläche dient. Das Gehäuse des Messsystems weist im Wesentlichen die Abmessungen und die äußere Form einer Computer-Mouse auf, über deren Auflagefläche für die Hand der zumindest eine Fingersensor hinausragt.

Ein Messverfahren unter Verwendung eines plethysmographischen System und eines Druckerzeugungssystems das mit Hilfe des plethysmographischen Systems geregelt wird, zeichnet sich dadurch aus, dass der Finger oder die Finger, die zur Messung herangezogen werden, in die aufblasbare Manschette eingeführt werden und zur Entspannung der Hand mit den angrenzenden Bereichen der Handinnenfläche auf eine Auflagefläche eines Gehäuses aufgelegt werden, das das Druckerzeugungssystem in sich aufnimmt.

Die Konfiguration kombiniert somit den Fingersensor, im konkreten die Fingermanschette und deren Lichterzeugungssystem mit dem Druckerzeugungssystem, so dass die vorhin angeführten Nachteile überwunden werden. Die Finger an denen gemessen wird bzw. die dazugehörige Hand rastet dabei auf einer Auflagefläche unter der das Druckerzeugungssystem angebracht wird. Eine oder mehrere Fingersensoren, also die Fingermanschetten inklusive dem PPG-System, werden auf der Handauflagefläche angebracht. Der Unterarm inklusive Handrücken ist somit frei und kann für intra-venöse oder intra-arterielle Zugänge verwendet werden. Weil die Finger, an denen gemessen wird mit deren Hand auf der Auflagefläche rasten können, werden Bewegungsartefakte reduziert. Ein Verdrehen oder Verkippen der Sensoren ist so nur mehr schwer möglich, weil so der Sitz der Sensoren und somit die Ankoppelung von Licht und Druck optimiert wird.

Weil die Hand auf der Auflagefläche ähnlich wie bei einer Computermaus rastet, wird das System auch CNAP-Mouse (Continuous Non-invasive Arterial Pressure) genannt. Im Körper bzw. dem Gehäuse der "Mouse" befindet sich zumindest das Druckerzeugungssystem für die Vascular Unloading Technique. Die "Ohren" der CNAP-Mouse stellen die - in der Regel zwei - Fingersensoren (Manschetten inklusive dem PPG-System) dar. Es werden deswegen zwei Fingersensoren verwendet, um eine längere Messdauer durch Abwechseln der Finger zu gewährleisten; an einem Finger wird der kontinuierliche Blutdruck bestimmt, während der andere Finger rastet. Naturgemäß funktioniert die CNAP-Mouse aber auch mit nur einem "Ohr", d.h. mit nur einem Fingersensor.

Die Erfindung wird im Folgenden anhand von teils schematisch dargestellten, beispielhaften Ausführungsvarianten näher erläutert:

Fig. 1 zeigt ein Messsystem gemäß Stand der Technik, bei dem das Druckerzeugungssystem am distalen Ende des Unterarmes angebracht ist;

Fig. 2 zeigt das erfindungsgemäße Messsystem in Form einer Computer-Mouse (CNAP-Mouse) in einer dreidimensionalen Darstellung;

Fig. 3 zeigt das Messsystem gemäß Fig. 2 in einer Explosionsdarstellung;

Fig. 4 zeigt das Messsystem gemäß Fig. 2 mit abgenommenem Manschettenteil und sichtbaren PPG-Elementen;

Fig. 5 zeigt in einer Draufsicht den symmetrischen Aufbau des Messsystems gemäß Fig. 2;

Fig. 6 zeigt alle möglichen Anwendungsvarianten, wie das Messsystem an der rechten oder linken Hand eines Patienten angebracht werden kann; sowie

Fig. 7 zeigt eine Ausführungsvariante des erfindungsgemäßen Messsystems in einer Explosionsdarstellung.

Die Beschreibung beschreibt ein Messsystem zur kontinuierlichen nicht-invasiven Bestimmung des Blutdruckes. Im Detail wird die Kombination des Druckerzeugungssystems mit den Fingersensoren bestehend aus Manschette und Lichtsystem - dem photoplethysmographisches System - dargelegt.

Bei der Vascular Unloading Technique wird grundsätzlich der intra-arterielle Druck in der Fingermanschette in Echtzeit nachgebildet. Dies geschieht mittels eines Regelungssystems, das als Eingangssignal zumindest das Lichtsignal aus dem PPG-System v(t) benötigt. Bei neuartigeren Regelsystemen, wie sie z.B. in den Schriften WO 2000/059369, WO 2005/037097, WO 2011/051819 oder WO 2011/051822 beschrieben wurden wird auch der Manschettendruck $p_c(t)$ als Eingangsvariable verwendet.

Grundsätzlich gilt folgende Gleichung für die Drücke, die beim Anlegen einer mit Luft gefüllten Manschette entsteht:

$$p_c(t) = p_a(t) - p_t(t) \qquad (1)$$

wobei $p_t(t)$ der sogenannte "Transmurale Druck" ist, also die Druckdifferenz zwischen Manschettendruck $p_c(t)$ und intra-arteriellen Blutdruck $p_a(t)$.

Dieser transmurale Druck $p_t(t)$ wirkt auf den Durchmesser der Arterie und dieser kann indirekt durch das PPG-Signal bzw. Volumenssignal v(t) bestimmt werden. Das Regelsystem verändert wie bereits beschrieben in Echtzeit den Manschettendruck $p_c(t)$ in der Art, dass v(t) konstant bleibt, bzw. dass $\Delta v(t) = 0$ ist. Ist nun diese Bedingung $\Delta v(t) = 0$ gegeben, dann ist auch der transmurale Druck $p_t(t) = 0$ und somit gilt

$$p_c(t) = p_a(t) - 0 \qquad (2)$$

Damit diese Echtzeitbedingung gilt, muss das Messsystem den Änderungen des intra-arteriellen Blutdruckes $p_a(t)$ folgen können. Betrachtet man die Regelungsbedingung im Frequenzbereich, so muss somit das Messsystem in der Lage sein Druckänderungen abzubilden, die über die obere Grenzfrequenz des intra-arteriellen Blutdruckes hinausgehen. Die obere Grenzfrequenz des intra-arteriellen Blutdruckes $p_a(t)$ liegt bei etwa 20Hz.

Für alle beschriebenen Verfahren und Geräte gilt also, dass der Manschettendruck $p_c(t)$ im gesamten relevanten Frequenzbereich mit einer oberen Grenzfrequenz von mindestens 20Hz gebildet werden muss, was eine Herausforderung für das Druckerzeugungssystem und dessen Ventil bzw. Ventilsystem ist. Es ist wichtig, dass das Druckerzeugungssystem und dessen Ventil bzw. Ventilsystem in unmittelbarer Nähe der Fingermanschette ist. Je nach der Steifigkeit des Anschlussschlauches liegt die maximale Entfernung bei ca. 30 - 50 cm.

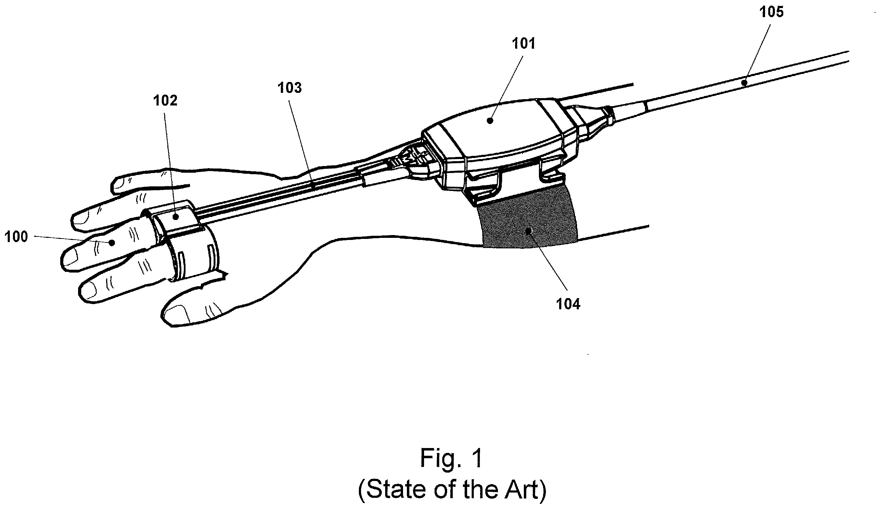

Fig. 1 zeigt, wie bei den Messsystemen gemäß dem Stand der Technik das Drucksystem 101 mit dem Fingersensor 102 verbunden ist. Der dargestellte Fingersensor 102 ist als Doppelfingersensor an Zeige- und Mittelfinger 100 der rechten Hand ausgeführt, um eine längere Messdauer zu gewährleisten. Der Fingersensor 102 wird durch ein Doppelschlauchsystem 103 mit Druck und Elektrik versorgt. Das Drucksystem 101 wird mittels Befestigung 104 am distalen Ende des Unterarmes angebracht und mit einem Kabel 105 elektrisch sowie mit dem Basisdruck versorgt.

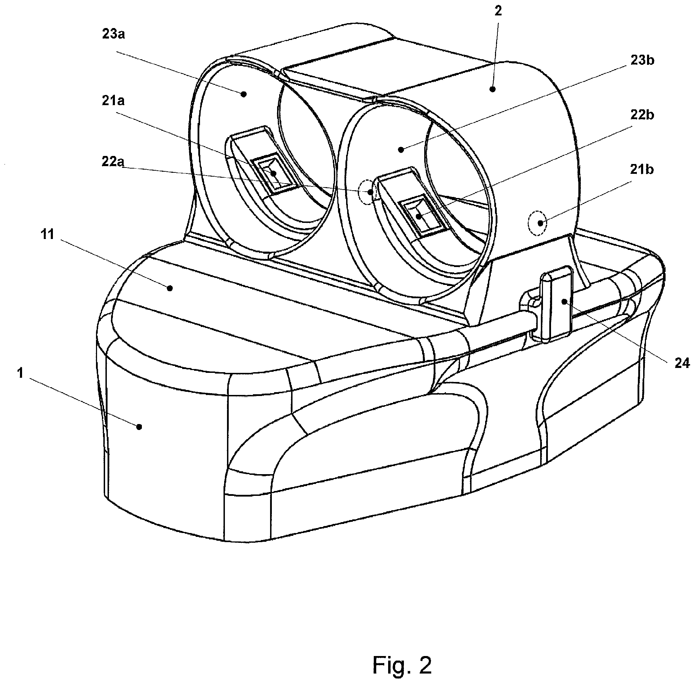

Fig. 2 zeigt das erfindungsgemäße Messsystem, die zusammengebaute CNAP-Mouse: Am Körper bzw. dem Gehäuse 1 der CNAP-Mouse befindet sich an der Oberfläche eine relativ große Auflagefläche 11, auf der die Hand im Messbetrieb rasten kann. Auf das Gehäuse 1 werden die Fingersensoren 2 montiert. Im gegenständlichen Fall wird ein Doppelfingersensor gezeigt, bei dem abwechselnd an einem Finger gemessen werden kann. Der Doppelsensor weist zwei Lichtquellen (LEDs) 21a, 21b auf sowie aus mindestens zwei Lichtsensoren 22a, 22b (verdeckt). Die beiden aufblasbaren Manschetten 23a und 23b sind auf den Innenflächen 23 des Fingersensors 2 angeordnet. Der die aufblasbaren Manschetten 23a, 23b aufnehmende Teil des Doppelfingersensors 2 ist abnehmbar und auswechselbar ausgeführt und wird durch die Arretierung 24 an einem Rastelement des Gehäuses 1 befestigt.

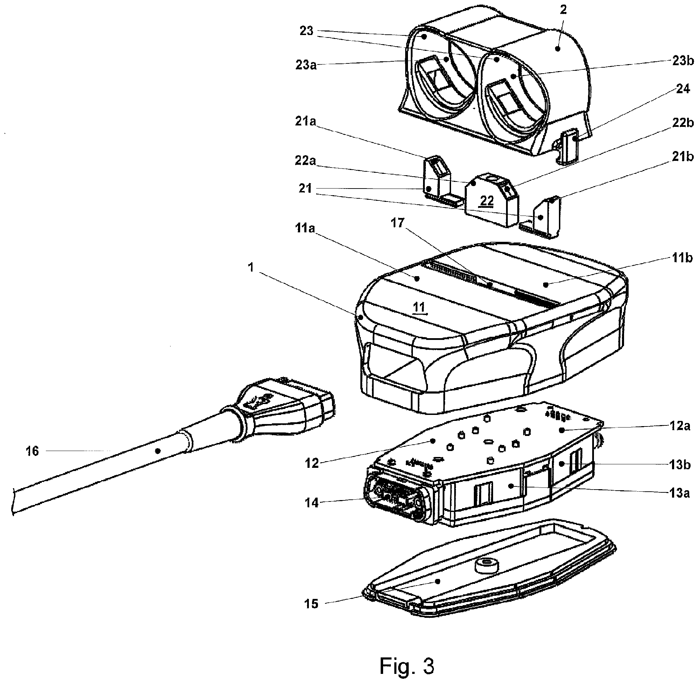

Fig. 3 zeigt eine Explosionsdarstellung der CNAP-Mouse: Zunächst wird gezeigt, wie sich das Druckerzeugungssystem 12 in den Körper bzw. in das Gehäuse 1 der Mouse unterhalb der Auflagefläche 11 für die Hand integriert. Das Druckerzeugungssystem 12 besteht in diesem Fall aus einer Elektronikplatine 12a, auf die neben den elektronischen Elementen für die Ansteuerung auch ein oder mehrere Ventile 13a, 13b, angebracht sind. Weiters ist vorzugsweise ein Stecker 14 auf der Platine 12a angebracht. Das Druckerzeugungssystem 12 wird im Gehäuse 1 untergebracht und mit einem Deckel 15 an der Unterseite des Gehäuses 1 gesichert. Ein Kabel 16 versorgt das Druckerzeugungssystem 12 mit dem Basisdruck sowie mit elektrischer Energie und den Signalen, die vom Regelungssystem für die Vascular Unloading Technique kommen.

An der Oberseite des Gehäuses 1 befinden sich Anschlüsse für einen oder mehrere Fingersensoren. Diese müssen einerseits elektrische Verbindungen für die Erzeugung des PPG-Signales aufweisen, andererseits müssen Druckanschlüsse für den Aufbau des Druckes in der einen oder mehreren Manschetten vorhanden sein. In der dargestellten Ausprägungsform, die das schon erwähnte Doppelfingersystem beschreibt, ist dies beispielsweise so gelöst: Ein Schlitz 17 bzw. eine Aufnahme unterteilt die Oberseite des Körpers der Mouse 1, die auch gleichzeitig die Auflagefläche 11 ist, in zwei Teilflächen 11a und 11b. In diesen Schlitz werden zwei äußere, verschiebbare Elemente 21 und ein zentrales Element 22 platziert. Die verschiebbaren Elemente 21 beinhalten die beiden LEDs 21a und 21b, die für die PPG-Signalerzeugung notwendig sind. Die äußeren Elemente 21 sind beweglich aufgeführt, um verschiedene Größen des Fingersensors aufzunehmen. Das Element 22 befindet sich in der Mitte des Schlitzes und ist nicht beweglich. Es beherbergt die beiden Lichtaufnehmer 22a und 22b.

Auf diesen Körper bzw. das Gehäuse 1 der Mouse mit den dargestellten Elementen 21, 22 für das PPG-Signal kann nun der abnehmbare Teil des Fingersensors 2 aufgesteckt werden. Diese Fingersensoren können unterschiedliche Größe bzw. Durchmesser aufweisen. So kann der optimale Sitz der Sensoren an die tatsächliche Fingergröße angepasst werden. Die Sensoren besitzen eine mit Luft befüllbare, aufblasbare Manschette 23a, 23b, deren Druck vom Druckerzeugungssystem 12 mit der erforderlichen Genauigkeit gesteuert werden kann. Dazu sind Anschlusselemente vorhanden, die im Element 22 integriert sind (nicht explizit dargestellt). Weiters muss das PPG-System in diese Sensoren integriert werden. Im gegenständlichen Fall ist dies wiederum mit dem bereits bekannten Doppelfingersensor 2 gelöst. Im Doppelfingersensor 2 befindet sich für jeden Finger eine Manschette 23a bzw. 23b an der Innenfläche 23. Der Vorteil dieser erfinderischen Ausführung besteht darin, dass für den abnehmbaren Teil des Doppelfingersensors 2, der die Manschetten 23a, 23b aufnimmt, keine elektrischen Versorgungen für das PPG-System notwendig sind. Die PPG-Elemente befinden sich auf dem Gehäuse 1 der Mouse und werden dort elektrisch versorgt.

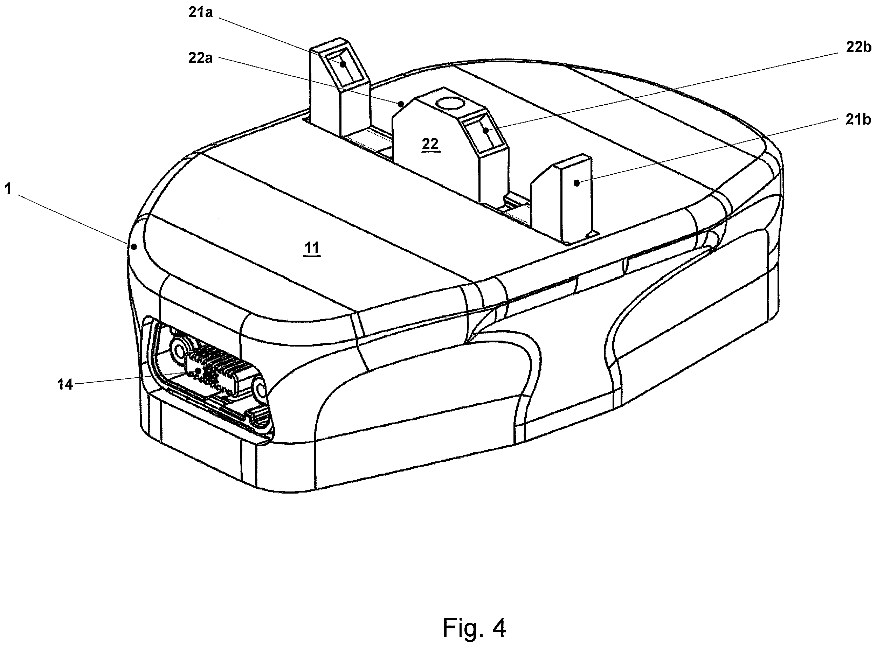

Fig. 4 zeigt wie die PPG-Elemente 21a, 21b und 22a, 22b auf dem Gehäuse 1 der Mouse angebracht sind. Man erkennt, dass die LED-Elemente 21a und 21b beweglich sind um den abnehmbaren Teil unterschiedlich großer Fingersensoren mit aufnehmen zu können. Das Element 22 mit den Lichtaufnehmern 22a und 22b ist hingegen fix in der Mitte der Auflagefläche 11 platziert. Der Stecker 14 versorgt in dieser Ausführungsform die CNAP-Mouse mit dem Basisluftdruck, elektrischer Energie und den Steuersignalen.

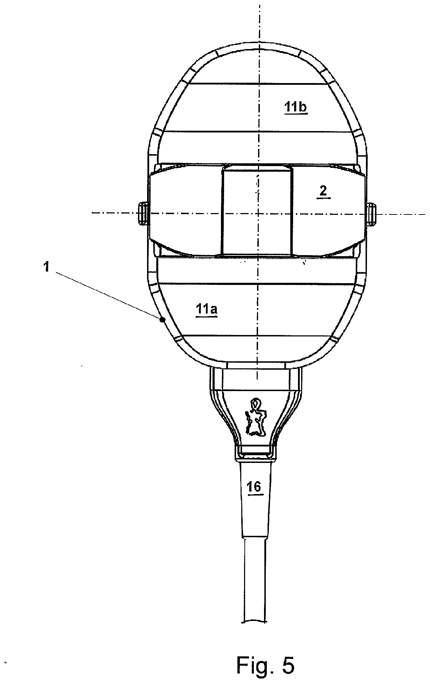

Fig. 5 zeigt eine spiegelsymmetrische Ausführungsform der CNAP-Mouse in der Draufsicht. Auf dem Gehäuse 1 der Mouse wird der Fingersensor 2 angebracht, der die Auflagefläche 11 für die Hand in zwei Teilflächen 11a und 11b teilt. Die CNAP-Mouse wird über das Kabel 16 mit Basisluftdruck, elektrischer Energie und den Steuersignalen von unten (in der Darstellung) bzw. proximal der Hand versorgt. Die Mouse kann problemlos umgedreht und auch von oben bzw. distal der Hand versorgt werden.

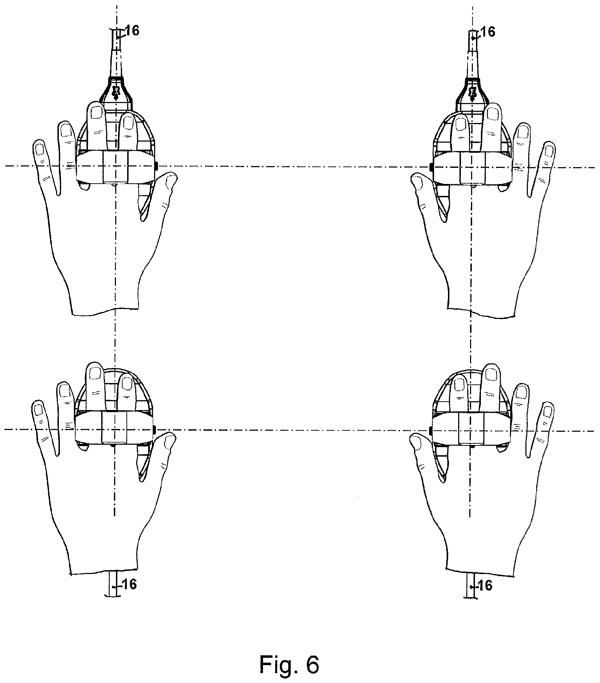

Fig. 6 zeigt den Vorteil dieser spiegelsymmetrischen Ausführungsform der CNAP-Mouse. Die Mouse lässt sich zunächst sowohl auf den Fingern der linken als auch auf den Fingern der rechten Hand anbringen. Dabei eignen sich sowohl die Paare Zeigefinger und Mittelfinger (dargestellt) als auch Mittelfinger und Ringfinger (nicht dargestellt). Dabei kann die Mouse durch das Kabel 16 von hinten (proximal der Hand) als auch von vorne (distal der Hand) versorgt werden. Das bietet dem Anwender höchstmögliche Flexibilität beim Anbringen des Gerätes am Patienten.

Die Auflagefläche für die Hand sollte vorzugsweise weich sein, damit sich Hand und Finger entspannen können um Bewegungsartefakte zu vermeiden. Dabei kann um bzw. auf die Auflagefläche 11 z.B. ein Lagerungskissen oder ein sogenanntes Gelpack platziert werden.

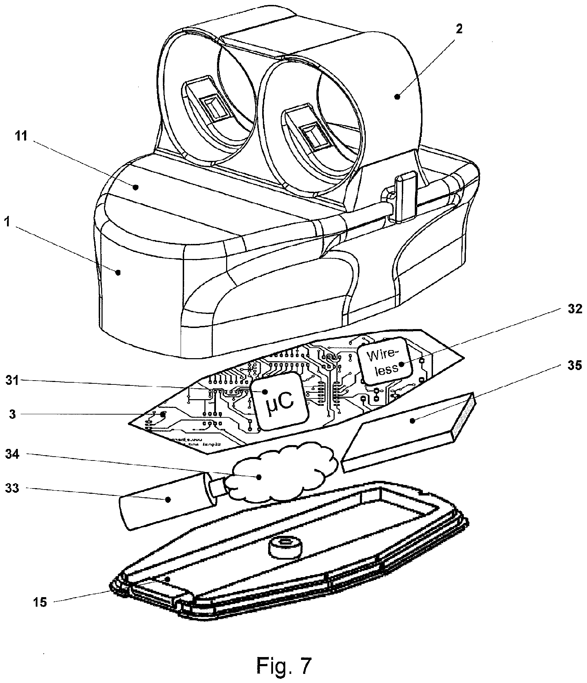

Weiters ist es von Vorteil, wenn sich auch das Regelungssystem 3 mit dem Microcontroller 31 für die Bestimmung der Echtzeitbedingung der Vascular Unloading Technique im Gehäuse 1 der CNAP-Mouse befindet (siehe Fig. 7). Beispielsweise kann man die Elektronikplatine des Regelungssystems 3 unterhalb des Druckerzeugungssystems 12 integrieren. Das resultierende Messsystem benötigt dann nur mehr eine Versorgung mit dem Basisdruck und der elektrischen Energie.

Konsequenterweise kann man auch eine Miniaturpumpe 33 in das Gehäuse 1 der Mouse integrieren. Zwischen der Pumpe 33 und dem Druckerzeugungssystem 12 wird meistens ein Luftreservoir 34 angeordnet, um hochfrequente Druckschwankungen, die von Unregelmäßigkeiten des Pumpenmotors resultieren, auszugleichen. Auch dieses kleine Luftreservoir 34 kann gemäß einer Ausprägungsform im Körper der Mouse integriert werden (siehe Fig. 7).

Um Volumen im Gehäuse 1 der Mouse einzusparen, kann gemäß einer Ausführungsvariante der Erfindung das Gehäuse unterhalb der Auflagefläche für die Hand druckdicht ausgebildet sein und als Luftreservoir dienen. Die Elektronikplatinen befinden sich dann im Gehäuse mit erhöhtem Luftdruck, der sich aber nicht nachteilig auf die Elektronik auswirkt. Somit nimmt das Reservoir kein unnötiges Volumen ein. Das resultierende Messsystem benötigt somit nur mehr eine Versorgung mit elektrischer Energie.

In einer konsequenten Weiterentwicklung kann auch ein Akku oder eine Batterie 35 in das Gehäuse 1 integriert werden. Mittels drahtloser Übertragung der Signale mit Hilfe eines Funkchips (siehe Element 32 "Wireless" in Fig. 7), kann das Messsystem komplett autark funktionieren.

Es wäre prinzipiell auch möglich, nur einen einzelnen Fingersensor an einer entsprechenden Aufnahme der CNAP-Mouse anzubringen. Ebenso können zwei einzelne oder sogar mehrere Fingersensoren angebracht werden. Die in den Abbildungen dargestellte Ausführungsform scheint allerdings eine sehr praktikable Form zu sein, da durch das Doppelfingersystem lange Messzeiten möglich sind, indem abwechselnd der eine und danach der andere Finger zur Messung herangezogen wird.

Die angeführten Ausführungsformen stellen nur Beispiele dar. Die Erfindung ist in den Ansprüchen definiert.

**Patentansprüche**

1. Messsystem zur kontinuierlichen Bestimmung des intra-arteriellen Blutdruckes an zwei Fingern einer Hand, mit einem Doppelfingersensor **(2)**, mit aufblasbaren Manschetten **(23a, 23b)** für die zwei Finger und einem plethysmographischen System, mit

    zumindest zwei Lichtquellen **(21a, 21b)**, mit einer oder mehreren Wellenlängen und zumindest zwei Lichtaufnehmern **(22a, 22b)**,

    sowie mit einem Druckerzeugungssystem **(12)** mit zumindest einem mit Hilfe des plethysmographischen Systems in Echtzeit geregelten Ventil **(13a, 13b)**, das geeignet ist in den Manschetten **(23a, 23b)** einen Druck zu erzeugen, der im Wesentlichen dem intra-arteriellen Blutdruck im Finger entspricht, **dadurch gekennzeichnet, dass** das Messsystem ein Gehäuse **(1)** aufweist, das im Wesentlichen die Abmessungen und die äußere Form einer Computer-Mouse aufweist, mit einer Oberfläche, die als Auflagefläche **(11)** für die Finger und die angrenzenden Bereiche der Handinnenfläche dient, wobei der Doppelfingersensor **(2)** über die Auflagefläche **(11)** hinausragt und der die aufblasbaren Manschetten **(23a, 23b)** aufnehmende Teil des Doppelfingersensors **(2)** abnehmbar und auswechselbar befestigt ist.

2. Messsystem nach Anspruch 1, **dadurch gekennzeichnet, dass** das Druckerzeugungssystem **(12)** mit dem zumindest einen geregelten Ventil **(13a, 13b)** im Gehäuse **(1)** unterhalb der Auflagefläche **(11)** angeordnet ist.

3. Messsystem nach Anspruch 1 oder 2, **dadurch gekennzeichnet, dass** an der Oberfläche des Gehäuses **(1)** ein Schlitz **(17)** oder eine Aufnahme ausgebildet ist, der/die die Auflagefläche **(11)** im Wesentlichen spiegelsymmetrisch in Teilflächen **(11a, 11b)** teilt und Elemente **(21, 22)** zur Aufnahme der Lichtquellen **(21a, 21b)** und der Lichtaufnehmer **(22a, 22b)** aufweist.

4. Messsystem nach Anspruch 3, **dadurch gekennzeichnet, dass** zwei äußere Elemente **(21)** in Richtung eines zentralen Elementes **(22)** verschiebbar im Schlitz **(17)** oder der Aufnahme des Gehäuses **(1)** angeordnet sind, an welchen Elementen **(21, 22)** der die aufblasbaren Manschetten **(23a, 23b)** aufnehmende Teil des Doppelfingersensors **(2)** auswechselbar befestigt ist.

5. Messsystem nach Anspruch 4, **dadurch gekennzeichnet, dass** die äußeren, verschiebbaren Elemente **(21)** die Lichtquellen **(21a, 21b)**, vorzugsweise LED's, aufnehmen und das zentrale Element **(22)** die Lichtaufnehmer **(22a, 22b)** aufnimmt.

6. Messsystem nach einem der Ansprüche 1 bis 5, **dadurch gekennzeichnet, dass** ein Regelungssystem **(3)**, vorzugsweise mit Microcontroller **(31)**, im Gehäuse **(1)** unterhalb der Auflagefläche **(11)** angeordnet ist.

7. Messsystem nach einem der Ansprüche 1 bis 6, **dadurch gekennzeichnet, dass** eine Pumpe **(33)** sowie ein Luftreservoir **(34)** im Gehäuse **(1)** unterhalb der Auflagefläche **(11)** angeordnet sind.

8. Messsystem nach einem der Ansprüche 1 bis 7, **dadurch gekennzeichnet, dass** das Gehäuse **(1)** der Messsystems unterhalb der Auflagefläche **(11)** druckdicht ausgebildet ist und als Luftreservoir dient.

9. Messsystem nach einem der Ansprüche 1 bis 8, **dadurch gekennzeichnet, dass** eine Batterie oder ein Akkumulator **(34)** sowie Elemente **(32)** zur drahtlosen Signalübertragung im Gehäuse **(1)** unterhalb der Auflagefläche **(11)** angeordnet sind.

10. Verfahren zur kontinuierlichen Bestimmung des intra-arteriellen Blutdruckes an den Fingern einer Hand, mit Hilfe eines Messsystems nach einem der vorhergehenden Ansprüche, wobei der Druck in den Manschetten **(23a, 23b)** des Doppelfingersensors **(2)** mit Hilfe des Druckerzeugungssystems **(12)** durch das plethysmographischen System geregelt wird; die Finger, an denen gemessen wird, in die aufblasbaren Manschetten **(23a, 23b)** eingeführt werden und zur Entspannung der Hand mit dem angrenzenden Bereich der Handinnenfläche auf die Auflagefläche **(11)** des Gehäuses **(1)** aufgelegt werden, das das plethysmographischen System und das Druckerzeugungssystem (12) aufnimmt und im Wesentlichen die Abmessungen und die äußere Form einer Computer-Mouse aufweist.

11. Verfahren nach Anspruch 10, **dadurch gekennzeichnet, dass** der für das Druckerzeugungssystem **(12)** erforderliche Basisdruck von einer Druckquelle außerhalb des Gehäuses **(1)** mittels eines Schlauchsystems zugeführt wird.

12. Verfahren nach Anspruch 10, **dadurch gekennzeichnet, dass** der für das Druckerzeugungssystem **(12)** erforderliche Basisdruck innerhalb des Gehäuses **(1)** mittels eingebauter Pumpe **(33)** erzeugt wird.

13. Verfahren nach Anspruch 10, **dadurch gekennzeichnet dass** die Steuersignale für das Druckerzeugungssystem **12** außerhalb des Gehäuses **1** erzeugt und mittels Kabel zugeführt werden.

14. Verfahren nach Anspruch 10, **dadurch gekennzeichnet, dass** die Steuersignale für das Druckerzeugungssystem **(12)** innerhalb des Gehäuses **(1)** mit der Hilfe eines Regelungssystems **(3)** berechnet werden.

15. Verfahren nach einem der Ansprüche 10 bis 14, **dadurch gekennzeichnet, dass** der Doppelfingersensor **(2)** austauschbar auf der Auflagefläche **(11)** für die Hand platziert wird.


**Claims**

1. Measuring system for the continuous determination of intra-arterial blood pressure at two fingers of a hand, with a double-finger sensor (2), having inflatable cuffs (23a, 23b) for the two fingers, and

   a plethysmographic system having at least two light sources (21a, 21b), with one or more wavelengths, and at least two light detectors (22a, 22b)

   and having a pressure generating system (12) with at least one valve (13a, 13b) which is controlled in real time by means of the plethysmographic system and is suitable for generating pressure in the cuffs (23a, 23b), which pressure essentially corresponds to the intra-arterial blood pressure in the finger, **characterised in that** the measuring system has a housing (1) which substantially has the dimensions and the outer shape of a computer mouse, with a surface which serves as a supporting surface (11) for the fingers and the adjacent areas of the palm, wherein the double-finger sensor (2) protrudes beyond the supporting surface (11) and the part of the double-finger sensor (2) which accommodates the inflatable cuffs (23a, 23b) is fastened in a removable and exchangeable manner.

2. Measuring system according to claim 1, **characterised in that** the pressure generating system (12) with the at least one controlled valve (13a, 13b) is arranged in the housing (1) underneath the supporting surface (11).

3. Measuring system according to claim 1 or 2, **characterised in that** on the surface of the housing (1) a slot (17) or a receptable is formed, which partitions the supporting surface (11) essentially mirror-symmetrically into partial surfaces (11a, 11b) and contains elements (21, 22) for accommodating the light sources (21a, 21b) and the light detectors (22a, 22b).

4. Measuring system according to claim 3, **characterised in that** two outer elements (21) are disposed in the slot (17) or the receptacle of the housing (1) so as to be slideable in the direction of a central element (22), on which elements (21, 22) the part of the double-finger sensor (2) accommodating the inflatable cuffs (23a, 23b) is interchangeably mounted.

5. Measuring system according to claim 4, **characterised in that** the outer, slideable elements (21) accommodate the light sources (21a, 21b), preferably LEDs and the central element (22) accommodates the light detectors (22a, 22b).

6. Measuring system according to one of claims 1 to 5, **characterised in that** a control system (3), preferably a microcontroller (31), is arranged in the housing (1) underneath the supporting surface (11).

7. Measuring system according to one of claims 1 to 6, **characterised in that** a pump (33) and an air reservoir (34) are arranged in the housing (1) underneath the supporting surface (11).

8. Measuring system according to one of claims 1 to 7, **characterised in that** the housing (1) of the measuring system is designed air-tight underneath the supporting surface (11), and acts as an air reservoir.

9. Measuring system according to one of claims 1 to 8, **characterised in that** a battery or a power pack (34) and elements (32) for the wireless transmission of signals are arranged in the housing (1) underneath the supporting surface (11).

10. Method for the continuous determination of intra-arterial blood pressure at the fingers of a hand by means of a measuring system according to one of the preceding claims,
    wherein the pressure in the cuffs (23a, 23b) of the double-finger sensor (2) is controlled by means of the pressure generating system (12) by the plethysmographic system;
    the fingers which are used for measurement are introduced into the inflatable cuffs (23a, 23b) and are placed, in order to keep the hand relaxed, together with the adjacent area of the palm on the supporting surface (11) of the housing (1), which accommodates the plethysmographic system and the pressure generating system (12) and substantially has the dimensions and the outer shape of a computer mouse.

11. Method according to claim 10, **characterised in that** the basic pressure needed for the pressure generating system (12) is supplied via a tube system from a pressure source external to the housing (1).

12. Method according to claim 10, **characterised in that** the basic pressure needed for the pressure generating system (12) is generated inside the housing (1) by means of a built-in pump (33).

13. Method according to claim 10, **characterised in that** the control signals for the pressure generating system (12) are produced outside the housing (1) and are fed in via a cable.

14. Method according to claim 10, **characterised in that** the control signals for the pressure generating system (12) are computed inside the housing (1) by means of a control system (3).

15. Method according to any of claims 10 to 14, **characterised in that** the double-finger sensor (2) is exchangeably mounted on the supporting surface (11) for the hand.

**Revendications**

1. Système de mesure pour déterminer en continu la pression sanguine intra-artérielle sur deux doigts d'une main comprenant :

   - un capteur à double doigt (2) muni de manchons gonflables (23a, 23b) pour les deux doigts, et
   - un système de pléthysmographie ayant :

     * au moins deux sources lumineuses (21a, 21b) à une ou plusieurs longueurs d'onde, et
     * au moins deux photorécepteurs (22a, 22b) ainsi qu'un générateur de pression (12) ayant :
       ▪ au moins une soupape (13a, 13b) régulée en temps réel à l'aide du système de pléthysmographie pour générer une pression dans les manchons (23a, 23b) correspondant pratiquement à la pression intra-artérielle dans un doigt,

système de mesure **caractérisé en ce qu'**il comprend :

   - un boîtier (1) ayant pratiquement les dimensions et la forme extérieure d'une souris d'ordinateur, avec une surface servant de surface d'appui (11) pour les doigts et les régions adjacentes du creux de la main, le capteur à double doigt (2) dépassant de la surface d'appui (11) et la pièce du capteur à double doigt (2) recevant les manchons gonflables (23a, 23b) est fixée de façon amovible et interchangeable.

2. Système de mesure selon la revendication 1, **caractérisé en ce que** le générateur de pression (12) avec au moins une soupape régulée (13a, 13b) est logé dans le boîtier (1) sous la surface d'appui (11).

3. Système de mesure selon la revendication 1 ou 2, **caractérisé en ce que** la surface supérieure du boîtier (1) a une fente (17) ou un logement qui divise la surface d'appui (11) pratiquement selon une symétrie plane en des surfaces partielles (11a, 11b) et comporte des éléments (21, 22) pour recevoir les sources lumineuses (21a, 21b) et les photorécepteurs (22a, 22b).

4. Système de mesure selon la revendication 3, **caractérisé en ce que** deux éléments extérieurs (21) sont montés coulissants en direction d'un élément central (22) dans la fente (17) ou dans le logement du boîtier (1) auxquels est fixée de manière échangeable la pièce du capteur à double doigt (2) munie des manchons gonflables (23a, 23b).

5. Système de mesure selon la revendication 4, **caractérisé en ce que** les éléments extérieurs coulissants (21) reçoivent les sources lumineuses (21a, 21b), de préférence des led et l'élément central (22) reçoit les photorécepteurs (22a, 22b).

6. Système de mesure selon l'une des revendications 1 à 5, **caractérisé en ce qu'** un système de régulation (3) est prévu dans le boîtier (1) sur la surface d'appui (11), de préférence avec un microcontrôleur (31).

7. Système de mesure selon l'une des revendications 1 à 6, **caractérisé en ce qu'** une pompe (33) ainsi qu'un réservoir d'air (34) sont logés dans le boîtier (1) sous la surface d'appui (11).

8. Système de mesure selon l'une des revendications 1 à 7,
**caractérisé en ce que**
le boîtier (1) du système de mesure est étanche en pression sous la surface d'appui (11) et il sert de réservoir d'air.

9. Système de mesure selon l'une des revendications 1 à 8,
**caractérisé en ce qu'**
une batterie ou un accumulateur (34) ainsi que des éléments (32) ou le transmetteur sans fil de signaux sont logés dans le boîtier (1) sous la surface d'appui (11).

10. Procédé pour déterminer en continu la pression sanguine intraartérielle des doigts d'une main à l'aide d'un système de mesure selon l'une des revendications précédentes,
on règle la pression dans les manchons (23a, 23b) du capteur à double doigt (2) à l'aide du système générateur de pression (12) par le système de pléthysmographie,
on engage les doigts que l'on mesure dans les manchons gonflables (23a, 23b) et pour détendre la main avec la région adjacente du creux de la main, on la place sur la surface d'appui (11) du boîtier (1) qui reçoit le système de pléthysmographie et le générateur de pression (12) et a pratiquement les dimensions et la forme extérieure d'une souris d'ordinateur.

11. Procédé selon la revendication 10,
**caractérisé en ce que**
la pression de base nécessaire au générateur de pression (12) est fournie par une source de pression extérieure au boîtier (1) par un système de tuyaux.

12. Procédé selon la revendication 10,
**caractérisé en ce que**
la pression de base nécessaire au générateur de pression (12) est générée à l'intérieur du boîtier (1) à l'aide d'une pompe intégrée (33).

13. Procédé selon la revendication 10,
**caractérisé en ce que**
les signaux de commande du générateur de pression (12) sont générés à l'extérieur du boîtier (1) et sont transmis par un câble.

14. Procédé selon la revendication 10,
**caractérisé en ce que**
les signaux de commande du générateur de pression (12) sont calculés dans le boîtier (1) à l'aide d'un système de régulation (3).

15. Procédé selon l'une des revendications 10 à 14,
**caractérisé en ce que**
le capteur à double doigt (2) est installé de manière interchangeable sur la surface extérieure (11) pour la main.

Fig. 1
(State of the Art)

Fig. 2

Fig. 3

Fig. 4

Fig. 5

Fig. 6

EP 3 419 515 B1

15

Fig. 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4406289 A, Wesseling
- US 4524777 A, Kisioka
- US 4726382 A, Boehmer
- WO 2000059369 A, Fortin
- WO 2004086963 A, Skrabal
- WO 2005037097 A, Fortin
- WO 2010050798 A, Guelen
- WO 2011045138 A, Langewouters
- WO 2011051819 A, Fortin
- WO 2011051822 A, Fortin
- WO 2012032413 A, Huber
- AT 412613B
- US 5218966 A